Europäisches Patentamt

European Patent Office

Office européen des brevets

(19)

(11) Publication number: 0 371 010
A1

## (12) EUROPEAN PATENT APPLICATION

(21) Application number: 90200083.5

(51) Int. Cl.5: A61K 37/30, A61K 47/10

(22) Date of filing: 26.11.85

(30) Priority: 26.11.84 JP 248898/84

(43) Date of publication of application:
30.05.90 Bulletin 90/22

(60) Publication number of the earlier application in accordance with Art.76 EPC: 0 183 527

(84) Designated Contracting States:
DE FR GB

(71) Applicant: YAMANOUCHI PHARMACEUTICAL CO. LTD.
No. 3-11 Nihonbashi-Honcho, 2-chome
Chuo-ku
Tokyo(JP)

(72) Inventor: Kagatani, Seiya
1506-6-304 Sangamyo
Yaiza-shi, Shizuoka(JP)
Inventor: Hasumi, Shunji
No. 6-2, Surugadai 2-chome
Fujieda-shi, Shizuoka(JP)
Inventor: Sonobe, Takashi
13-8 Minamisurugadai 5-chome
Fujueda-shi, Shizuoka(JP)
Inventor: Aruga, Masayoshi
1-7, Hinode-cho
Shizuoka-shi, Shizuoka(JP)

(74) Representative: Geering, Keith Edwin et al
REDDIE & GROSE 16 Theobalds Road
London WC1X 8PL(GB)

(54) Absorbable calcitonin medicament.

(57) Calcitonin is a polypeptide hormone having various medical activities used for treatment of osteoporosis, hypercalcemia, Paget's disease etc. There is provided an intranasal medicament composition comprising [a] calcitonin and [b] at least one absorption enhancer selected from benzyl alcohol, ethanol, and polyethylene glycol 400 [Macrogol 400].

There is also provided means enabling application of the composition to the nasal mucosa. By the use of a specific absorption enhancer in the calcitonin internasal composition, efficiency of absorbing across the membrane of the nasal cavity is improved.

EP 0 371 010 A1

# ABSORBABLE CALCITONIN MEDICAMENT

The present invention relates to intranasal [i.e. for administration via the nasal route] medical compositions containing calcitonin as an active ingredient together with absorption enhancer.

Calcitonin is a polypeptide hormone having various medical activities used for treatment of osteoporosis, hypercalcemia, Paget's disease etc.

Calcitonin, like other pharmacologically active peptides, is decomposed in the gastrointestinal tract by digestive juice, so that oral administration is ineffective. Due to this, and to its poor absorption, calcitonin is generally administered by injection, but this can be so painful that self injection is unsuitable. Accordingly administration of calcitonin has been inconvenient and costly.

Recently, it has been found that nasal application of calcitonin gives effects similar to conventional muscular injection, and various intranasal calcitonin compositions have been proposed. However, a polypeptide of such large molecular weight as calcitonin has poor inherent nasal absorbability and therefore absorption enhancers, for example surface active agents, are generally incorporated [published Japanese Patent Application Nos.89619/84 and 130820/84]. In these proposals, both amphoteric and cationic surface active agents are employed, but it is said that nonionic surface active agents, inter alia polyoxyethylene lauryl ether, are particularly excellent as absorption accelerators. However, such an ether type surface active agent promotes the drug absorption by destroying the nasal membrane. Thus, the preferred surface active agent has a strong toxicity to tissue and so is unsatisfactory for practical use alone.

From extensive investigations we have found effective enhancers for the nasal absorption of calcitonin which are suitable for practical use. The present invention provides an intranasal medical composition comprising [a] calcitonin and [b] at least one absorption enhancer therefor selected from benzyl alcohol, ethanol,
and polyethylene glycol 400 [Macrogol 400].

As calcitonin for the present invention, various kinds such as salmon calcitonin, human calcitonin, elcatonin, porcine calcitonin, etc. can be used.

The absorption enhancers may be used singly or in combinations of two or more.

The intranasal medical composition of the present invention may be in the form of an aqueous solution, hydrogel or solid powder.

An aqueous solution can be prepared by dissolving calcitonin and absorption enhancer in water or a buffer solution in conventional manner. In this case, additive is added to and dissolved in the aqueous solution, if necessary. It is preferred that pH of the aqueous solution be between 3 and 5, for stability.

As the buffer, citrates, tartarate, malates, etc. can be employed, in a preferred pH range of 3 to 5.

As additive, one or more substances selected from sterilizers, preservatives, tackifiers, surface active agents, stabilisers, etc. conventionally used for intranasal agents can be incorporated.

Conventional steriliser and preservative may be used and examples include p-oxybenzoates, propylene glycol, benzetonium chloride, sorbic acid [Na], etc.

As tackifier, polyvinyl alcohol, polyvinyl pyrrolidone, dextran, etc. may be employed.

Surface active agent can be added as dispersing and emulsifying agent for various additives; nonionic surface active agents that cause little irritation to the nasal mucous membrane are preferred. As these nonionic surface active agents, for example, polyoxyethylene monostearate, polyoxyethylene sorbitan monooleate, polyoxyethylene-hydrogenated castor oil, etc. are used.

As stabiliser, mention may be made of gelatin or albumin.

The solution can be administered dropwise or by spraying using a dropping container, a sprayer or a nasal aerosol applicator.

For a powder composition, mannitol, inositol, glucose, etc. can be added in conventional manner; after dissolving and then freeze-drying, the resulting solid is pulverised into fine powder to be administered by the nasal route. Such powder may for example be packed in a capsule, the capsule set in a spraying device and penetrated by a needle to make pores at its top and bottom, and air applied [e.g. via a rubber bulb] to blow the powder out. When volatile liquid components such as ethanol, benzyl alcohol, etc. are used as the absorption enhancers, the powdery form is not suitable.

In the case of an aqueous gel, calcitonin can be formed into the aqueous gel using conventional gel bases, for example, natural gums, methyl celluloses, acrylic polymers, vinyl polymers, polysaccharides, etc.

The proportions of calcitonin as active ingredient, absorption enhancer and various additives to be used in the medical composition of the present invention are not particularly limited but are appropriately determined depending upon dosage form [solution, gel or powder, etc.]. In the case of an aqueous solution for intranasal drops, calcitonin is suitably formulated in a concentration of from 200 to 600 IU/ml, preferably 500 to 2000 IU/ml; the individual dose is preferably 0.05 to 0.2 ml and administered preferably 1 to 5 times daily. The amount of absorption enhancer incorporated varies, but in the case of an aqueous solution it is appropriately from 0.05 to 15% [w/v]; a particularly preferred range is 1.0 to 10% [w/v] for ethanol and 0.1 to 5% [w/v] for other absorption enhancers.

By the use of a specific absorption enhancer in the calcitonin intranasal agent of this invention, efficiency of absorbing across the membrane of the nasal cavity is improved.

The present invention is illustrated in more detail by the following Examples but is not to be limited thereto.

| Preparation Example 1 | |
|---|---|
| | per 1 ml |
| Salmon calcitonin | 350 IU |
| Citric acid hydrate | 12.2 mg |
| Sodium citrate | 12.4 mg |
| Absorption enhancer | [cf. Table 1] |

TABLE 1

| Absorption Enhancer | Amount [mg] |
|---|---|
| a Control | - |
| b Benzyl alcohol | 1.0 |
| c Benzyl alcohol | 10 |
| d Ethanol | 50 |
| e Macrogol 400 | 10 |
| f Sodium pyrophosphate | 30 |
| Benzyl alcohol | 10 |

700 IU salmon calcitonin was dissolved in less than 1 ml of a solution containing 24.4 mg citric acid. Absorption enhancer in an amount twice that shown in Table 1 was dissolved in less than 1 ml of a solution containing 24.8 mg sodium citrate. After pH adjustment to 4.0 using 1N aqueous hydrochloric acid or 1N aqeuous sodium hydroxide, water was added to make 1 ml of each solution. The equal volumes were mixed to provide 2 ml of the preparation having the required concentration. The salicylate preparation was a suspension. The sodium caprate preparation was adjusted to pH 8.

Example 1

Sprague Dawley strain male rats[115-145 g] fasted for 18 hours were anaesthetized with pentobarbital [50 mg/kg, intraperitoneal injection]. Aqueous calcitonin preparations [a] to [m] as in Preparation Example 1 were administered to the rats in a dose of 5 IU/kg.

Administration was performed by using a microsyringe [2 μl] connected with a polyethylene tube [PE 10, Clay Adams] and injecting about 2 μl at a distance of 5 to 6 mm from the nasal septum depending upon the body weight. Evaluation of absorption of the calcitonin preparation through the nasal membrane was performed by measuring the concentration of calcium in serum. The calcium concentration was quantitatively determined using a calcium meter [CA-30, Joko]. Rats were sacrificed prior to administration and 1, 2 and 3 hours after administration. Blood was collected from the descending large vein. The results are showin in Table 2. The data shown in Table 2 are mean values of 3 or more rats.

TABLE 2

| Concentration of Calcium in Serum after Intranasal Administration of Calcitonin [5 IU/kg] | | | | |
|---|---|---|---|---|
| Absorption Enhancer | Concentration % | Ca | mg | % |
| | | 1h | 2h | 3h |
| a Control | - | 10.65 | 10.24 | 10.81 |
| b Benzyl alcohol | 0.1 | 9.01 | - | 11.17 |
| c Benzyl alcohol | 1 | 8.41 | 10.53 | 10.73 |
| d Ethanol | 5 | 8.46 | 11.12 | 11.14 |
| e Macrogol 400 | 1 | 8.71 | 11.24 | 11.18 |
| f Sodium pyrophosphate | 3 | | | |
| Benzyl alcohol | 1 | - | 8.64 | - |

Calcium concentration in serum prior to administration: 10.67 mg%.

From Table 2, it is noted that Ca concentration in serum is significantly reduced by the addition of a specific absorption enhancer compared to the control.

In a manner similar to Preparation Example 1, calcitonin intranasal compositions shown in the following Preparation Examples were obtained. These calcitonin solutions provide reduction of the calcium concentration in serum as in Example 1.

| Preparation Example 2 | |
|---|---|
| Procine calcitonin | 1400 IU |
| Benzyl alcohol | 10 mg |
| Malic acid | 13.4 mg |
| Methyl p-oxybenzoate | 5 mg |
| Propyl p-oxybenzoate | 2 mg |

1N aqueous sodium hydroxide was added to adjust pH to 4.0. Water was added to make the final volume 1 ml.

| Preparation Example 3 | |
|---|---|
| Salmon calcitonin | 1400 IU |
| Macrogol 400 | 10 mg |
| Thiamine hydrochloride | 10 mg |
| Citric acid monohydrate | 12.2 mg |
| Sodium citrate | 12.4 mg |

Water was added to make the final volume 1 ml.

| Preparation Example 4 | |
|---|---|
| Salmon calcitonin | 14000 IU |
| Sodium pyrophosphate | 300 mg |
| Benzyl alcohol | 100 mg |
| Benzetonium chloride | 1 mg |
| Citric acid monohydrate | 122 mg |
| Sodium citrate | 124 mg |

5N hydrochloride acid was added to adjust pH to 4.0. Water was added to make the final volume 10 ml.

**Claims**

1. An intranasal medicament composition comprising [a] calcitonin and [b] at least one absorption enhancer selected from benzyl alcohol, ethanol,
and polyethylene glycol 400.

2. A container containing a composition as claimed in claim 1 and provided with means enabling application of the contained composition to the nasal mucosa.

3. A container according to claim 2 provided with means enabling application of the contained composition to the nasal mucosa in spray form.

4. An applicator device containing a composition as claimed in claim 1 and provided with means enabling application of the contained composition to the nasal mucosa in spray form.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | GB-A-2 142 335  (CIBA-GEIGY AG)<br>* Page 5, example 4 *<br>--- | 1-4 | A 61 K  37/30<br>A 61 K  47/10 |
| Y | EP-A-0 115 627  (ARMOUR PHARMACEUTICAL CO.)<br>* Page 10, example 9; claims * &<br>JP-A-59 130 820 (Cat, D,Y)<br>--- | 1-4 | |
| Y | JOURNAL OF PHARMACEUTICAL SCIENCES, vol. 73, no. 10, October 1984, pages 1366-1368; K. MORIMOTO et al.: "Enhanced rectal absorption of [Asu1,7]-eel calcitonin in rats using polyacrylic acid aqueous gel base"<br>* Page 1367, figure 4 *<br>--- | 1-4 | |
| Y | US-A-4 151 276  (CAULIN et al.)<br>* Column 4, lines 46-56; claim 8 *<br>----- | 1-4 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 27-02-1990 | BENZ K.F. |

EPO FORM 1503 03.82 (P0401)